(19) Europäisches Patentamt
European Patent Office
Office européen des brevets

(11) Publication number: **0 248 562 B1**

(12) **EUROPEAN PATENT SPECIFICATION**

(45) Date of publication of patent specification: **25.10.95**

(51) Int. Cl.[6]: **C07K 5/00**, A61K 38/55, C07D 207/16, A61K 31/16, A61K 31/40

(21) Application number: **87304451.5**

(22) Date of filing: **19.05.87**

(54) **Difluoro peptide compounds.**

(30) Priority: **05.06.86 GB 8613703**
**16.01.87 US 4020**

(43) Date of publication of application:
**09.12.87 Bulletin 87/50**

(45) Publication of the grant of the patent:
**25.10.95 Bulletin 95/43**

(84) Designated Contracting States:
**DE FR GB IT**

(56) References cited:

**BIOCHEMISTRY; vol. 24, no. 8, 9th april 1985, pages 1813-1817, The American Chemical Society, Washington, US; M. GELB at al.: "Flouro ketone inhibitors of hydrolytic enzymes"**

**TETRAHEDRON LETTERS, vol. 27, no.2, January 1986, pages 135-138, Pergamon Press Ltd, Oxford, GB; B. IMPERIALI et al.: "A versatile synthesis of peptidyl fluoromethyl ketones"**

(73) Proprietor: **ZENECA INC.**
**Concord Pike & New Murphy Road**
**Wilmington,**
**Delaware 19897 (US)**

(72) Inventor: **Trainor, Amy Diane**
**7 Marshall Road**
**Glenmills PA, 19342 (US)**
Inventor: **Stein, Mark Morris**
**1114 Grinnell Road**
**Wilmington DE 19803 (US)**

(74) Representative: **Smith, Stephen Collyer et al**
**Intellectual Property Department**
**ZENECA Pharmaceuticals**
**Mereside**
**Alderley Park**
**Macclesfield**
**Cheshire SK10 4TG (GB)**

**EP 0 248 562 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background and Summary of the Invention

The present invention relates to certain difluoro peptide compounds which are human leukocyte elastase (HLE) inhibitors and are also useful as research tools in pharmacological and related studies and in the treatment of tissue degenerative diseases such as pulmonary emphysema, atherosclerosis, rheumatoid arthritis and osteoarthritis in warm blooded animals. The invention also includes intermediates useful in the synthesis of these peptide derivatives, processes for preparing them, pharmaceutical compositions containing such peptide derivatives and methods for their use. Proline based peptide aldehydes are disclosed in European Patent Application 84302621.2. Fluoroketone inhibitors of hydrolytic enzymes are disclosed in Gelb, M.H. et al, Biochemistry (1985) 24, 1813-1817 for non-serine proteases. Imperiali, B. et al, Tetra. Letters (1986) 27, No. 2, 135-138 shows selected fluoromethyl ketones. Thaisrivongs, S. et al, J. Med. Chem. (1985) 28, No. 11, 1553-1555 discloses selected fluoro ketones as renin inhibitors.

### Description of the Invention

The substituted peptides of the present invention may be represented by the following formula Ib:

(Formula set out on pages following Examples) Ib

wherein $R^1$ is (1-3C) alkyl; $R^3$ is benzyl; $R^C$ is hydrogen; A is oxycarbonyl; and $R^B$ is selected from $-CH(CH_2C_6H_5)COOCH_3$, $-CH(CH_2C_6H_5)CONH_2$, $-CH(CH_2OH)CONH.CH(CH_2C_6H_5).(CONH_2)$, $-CH(CH_2C_6H_5)COOH$, $-CH_2COOCH_3$, $-CH_2COOH$, $-CH(CH_2C_6H_5)CONH.CH_2COOCH_3$, $-CH(CH_2C_6H_5)CONH.CH_2COOH$, $-CH[(CH_2)_4NHOCOCH_2C_6H_5]COOCH_2C_6H_5$, $-CH[(CH_2)_4NHOCOCH_2C_6H_5]COOH$, $-CH[(CH_2)_2OCOCH_2C_6H_5]COOCH_2C_6H_5$, $-CH(CH_2C_6H_5)COOCH_2C_6H_5$ and $-CH_2COOCH_2C_6H_5$; or a pharmaceutically acceptable salt thereof.

More particular values for compounds of the invention include those formed with the following members of the groups defined above:

$R^1$ is especially isopropyl;

Still more particular values for the groups defined above when compounds of formula Ib are selected are as follows: $R^1$: isopropyl; $R^3$: benzyl; A: OCO; $R^C$: H.

In compounds of formula Ib, when $R^B$ is formula IIa, particular values for $R^6$, $R^7$ and $R^8$ are: $R^6$: $CH_2OH$, $ØCH_2$; $R^7$: H, $ØCH_2$; $R^8$: $NH_2$, $OCH_3$, OH.

In compounds of formula Ib, when $R^B$ is formula IIb, particular values for $R^6$ and $R^8$ are: $R^6$: $CH_2Ø$, $CH_2OH$, H; $R^8$: $NH_2$, $OCH_3$, OH, $OCH_2Ø$.

The title compounds of Examples 11, 13 and 14 are especially preferred.

The salts of the compounds of formula Ib include pharmaceutically acceptable base or acid addition salts such as those made with a mineral acid, e.g., hydrochloric, or an organic acid such as citric, maleic, fumaric or acetic. Base-addition salts include those made with alkali metal hydroxides such as sodium hydroxide, alkali metal carbonates and bicarbonates, alkaline earth hydroxides and organic amine salts. Such salts may be prepared by dissolving the peptide derivative in a mixture of water and a water-miscible organic solvent, adding an aqueous solution of the base and recovering the salt from the aqueous solution.

The preferred compounds of the present invention are of the S configuration (i.e., that of the naturally occurring L-amino acids) at chiral centers identified by * in formula, IIIb below (or identified by * in formulae IIId and IIIe for specific values of $R^B$) and the methods of synthesis described below provide isomers with the S configuration at the chiral center identified by symbol # or isomeric mixtures as a result of the R and S configurations at the chiral center identified by the symbol #. It is generally preferred that the compounds have the S configuration at the center identified by the symbol #. (Note that Formulae IIId and IIIe correspond to groups IIa and IIb).

(Formula set out on pages following Examples) IIIb

(Formula set out on pages following Examples) IIId

(Formula set out on pages following Examples) IIIe

As will be appreciated by those skilled in the art, the activity of the individual isomers is not the same, and it is therefore preferred to utilize the more active isomer. The present invention includes compounds resulting from the S and/or R configuration at the chiral center labelled #.

$R^B$, may have chiral centers. The present invention includes compounds of formula, Ib wherein the chiral centers included in, $R^B$ are of the S and/or R configurations.

As will be appreciated by those skilled in the art, the difluoro ketone derivatives can exist as solvates, particularly hydrates, formula IVb, and these are encompassed by the present invention.

(Formula set out on pages following Examples) IVb

According to a further feature of the invention there are provided pharmaceutical compositions comprising a pharmaceutically effective amount of at least one peptide derivative of formula Ib and a pharmaceutically acceptable diluent or carrier.

The difluoro peptide compounds of formulae Ib may be prepared as follows.

Method A

A compound of formula, Ib may be prepared from the corresponding alcohol of formula VIIIb:

(Formula set out on pages following Examples) VIIIb

by an oxidative process. Methods which are useful include the use of oxalyl chloride, DMSO and a tertiary amine (see Marx, M., et al., J. Org. Chem., (1984) 49, 788-793, with the best results being obtained with 10-20 equivalents of oxidizing agent), the use of acetic anhydride and DMSO, the use of chromium trioxide pyridine complex in methylene chloride, and the use of Dess-Martin periodinane [1,1,1-triacetoxy-2, 1-benzoxiodol-3(3H)-one] (method of Dess, D.B. et al., J. Org. Chem., (1983) 48, 4155-56).

An alcohol of formula VIIIb may be prepared from the corresponding aldehyde of formula VIb:

(Formula set out on pages following Examples) VIb

by reacting the aldehyde with ethyl 2-bromo-2,2-difluoroacetate (obtained from SCM, Specialty Chemicals) and Zn in refluxing THF (see Hallinan, E.A., et al., Tetrahedron Letters, (1984) 25 (#22), 2301-2302) to give a compound of the following formula VIIb,

(Formula set out on pages following Examples) VIIb

which in turn may be reacted with an amine of formula V:

(Formula set out on pages following Examples) V

and tetramethylguanidine and $CHCl_3$ at 0 °C to room temperature to afford the particular compound of formula VIIIb. (See R. H. Abeles, et al., Biochemistry, (1985) 24, 1813).

An aldehyde of formula VIb may be prepared by oxidation of the corresponding alcohol of formula XIVb:

(Formula set out on pages following Examples) XIVb

(e.g., oxidation conditions as described in M. Marx, et al., J. Org. Chem., (1984) 49, 788-793), or by hydrolysis or transacetalization of the corresponding acetal of formula XVb:

(Formula set out on pages following Examples) XVb

See, for example, the preparation of such compounds as described in European Patent Application 84302621.2(EP-A-0124317).

A compound of formula XIVb (for use in making compounds of formula VIb) may be prepared by reacting an amino alcohol of formula XVI:

(Formula set out on pages following Examples) XVI

with an appropriate free acid of formula XVIIb

(Formula set out on pages following Examples) XVIIb

by standard peptide coupling procedures using methods commonly known to those skilled in the art, such as those described in M. Bodanszky, Principles of Peptide Synthesis, Springer-Verlag, Berlin, (1984), and The Peptides. Analysis, Synthesis and Biology (ed. E. Gross and J. Meinhofer), Vols. 1-5, (Academic Press, New York) 1979-1983. A compound of formula XVIIb may be prepared by standard peptide coupling and deprotection procedures as described above. The amino alcohols of formula XVI (when not commercially available) may be prepared from the corresponding alpha-amino acids of the formula $H_2NCHR^1COOH$ by reaction with a reducing agent such as diborane. See U.S. Patent 3,935,280 to Lane.

A compound of formula XVb may be prepared by reacting an amino acetal of formula XVIIIa:

(Formula set out on pages following Examples) XVIIIa

with an appropriate acid of formula XVIIb by standard peptide coupling procedures; The amino acetal of formula XVIIIa can be prepared as described in Examples (1a-1d).

An acetal of formula XVb may be prepared, when appropriate, by an acid catalyzed acetalization of a compound of formula VIb (e.g., with triethylorthoformate in absolute ethanol acidified with p-toluenesulfonic acid at room temperature).

An alternate method for the preparation of a compound of formula VIIIb from a compound of formula VIIb, comprises reacting a compound of formula VIIb with about 1.25 equivalents of 1N NaOH in $CH_3OH$ to give a corresponding compound of formula IXb,

(Formula set out on pages following Examples) IXb

followed by reacting a compound of formula IXb with a compound of formula V:

(Formula set out on pages following Examples) V

using HOBT, WSCDI and THF.

Amines of formula V are generally commercially available or may be prepared by standard techniques of organic chemistry, including standard peptide coupling and protection procedures as referenced above, and by analogy with the synthesis of known, structurally similar compounds.

Method B

A compound of formula, Ib may be prepared from the corresponding acid of formula XXVIIb:

(Formula set out on pages following Examples) XXVIIb

by coupling the acid with an amine of formula V (using similar conditions to those described above for the conversion of IXb to VIIIb) to afford the corresponding compound of formula Ib. A starting acid of formula, XXVIIb may be obtained, for example, by hydrolyzing the corresponding ester such as the ethyl ester of formula XXVIb:

(Formula set out on pages following Examples) XXVIb

using a similar method to that described above for the hydrolysis of a compound of formula VIIb to a compound of formula IXb. A starting ester of formula XXVIb may be obtained, for example, by oxidation of the corresponding alcohol of formula VIIb using one of the oxidation methods described in Method A.

(Formula set out on pages following Examples) VIIb

Method C

A compound of formula Ib where $R^B$, contains a carboxy group may be prepared by decomposing the ester group of a corresponding compound of formula Ib where $R^B$ contains an ester group. The ester groups contained by $R^B$ of compounds of formula, Ib described above include aryloxycarbonyl wherein the aryl group contains 6, 10 or 12 carbons, aralkoxycarbonyl wherein the aralkoxy group contains from 7 to 13 carbons, and alkoxycarbonyl wherein the particular alkoxy group contains from 1 to 3, 1-4, 1-5 or 1-6 carbons, as well as those resulting when $R^8$ is (1-3C)alkoxy, (7-12C)aralkoxy and (6C)aryloxy.

It will be appreciated that the decomposition can be performed using any one of a variety of procedures well known in the art of organic chemistry. Thus, it may be carried out, for example, by conventional hydrolysis under acid or base conditions, adjusted as necessary to minimize any hydrolytic removal of other functional groups in the molecule. Also, when the ester is an aralkoxycarbonyl group, such as benzyloxycarbonyl, it may be possible to carry out the decomposition by reductive means, for example, by the use of hydrogen at about atmospheric pressure in the presence of a suitable catalyst, such as palladium or platinum, conveniently on charcoal as a support.

A preferred method for decomposing an ester of formula Ib comprises reacting the ester with a suitable base, for example, an alkali or alkaline earth metal hydroxide or carbonate (such as lithium hydroxide, potassium hydroxide, sodium hydroxide, calcium hydroxide or potassium carbonate) in a suitable, aqueous solvent or diluent, for example, water, optionally together with a water-miscible organic cosolvent, such as methanol, and conveniently at or near ambient temperature. When such a method is employed, the resultant carboxylic acid of formula, Ib where $R^B$ contains a carboxy group is initially obtained as the corresponding salt of the base used for the hydrolysis and may be isolated as such or converted to the free acid form by a conventional acidification procedure.

As will be apparent to one skilled in the art, it may be desired to convert a compound of formula, Ib into another compound of formula, Ib using a standard method well known in the art, such as, for example, conversion of an acid or ester into the corresponding amide.

Also, it may be desired to optionally use a protecting group during all or portions of the above described processes; the protecting group may then be removed when the final compound is to be formed. (See Greene, T.W., Protective Groups in Organic Synthesis, Wiley-Interscience, New York (1981).)

When a pharmaceutically acceptable salt is desired or required, it may be obtained using standard procedures well known in the art, for example, by further reacting a suitably acidic compound of formula, Ib with a suitable base affording a physiologically acceptable cation or by further reacting a sufficiently basic compound of formula, Ib with a suitable acid affording a physiologically acceptable anion.

Inhibition Measurements:

The ability of compounds of the invention to act as elastase inhibitors may be initially determined by the ability of a compound of the invention to inhibit the action of human leukocyte elastase (HLE) on a low molecular weight peptide substrate. The potency of an inhibitor is evaluated by obtaining a kinetic

determination of the dissociation constant, $K_i$, of the complex formed from the interaction of the inhibitor with HLE. The substrate used was the anilide methoxysuccinyl-L-alanyl-L-alanyl-L-prolyl-L-valine-p-nitroanilide as described by K. Nakajima et al. in J. Biol. Chem., (1979) 254, 4027-4032 and by T. Teshima et al. in J. Biol. Chem., (1982) 257, 9, 5085-5091. The HLE enzyme used in these studies may be obtained from Elastin Products of St. Louis, Missouri or can be purified according to B. R. Viscarello et al. in Preparative Biochemistry, (1983) 13, 57-67 as follows, all work being done in a cold room at 4°C.

Salt Extraction-DNase Treatment: The starting material, 193 g of purulent sputum, was homogenized with 200 ml of cold distilled water and centrifuged at 30,000 x gravity for 20 min. at 4°C. The supernatant was discarded and the pellet extracted with high salt and treated with DNase as per the method of D. Y. Twumasi et al. in J. Biol. Chem., (1977) 252, 1917-1926. Chromatography on Elastin Agarose: The precipitate from the DNase digest was taken up in two 40 ml portions of 50 mM Tris, 1.0 M NaCl, pH 8; the suspension was centrifuged and the resulting supernatant applied directly to a column of soluble elastin-Sepharose 4B (2.5 x 20 cm). The column was washed with equilibrating buffer (50 mM Tris, 50 mM NaCl, pH 8.0) until the optical density at 280 nm ($OD_{280}$) of the eluate returned to baseline. Additional contaminating protein was eluted with two column volumes of 50 mM acetate, 1.0 M NaCl, pH 5.0. Elastase and cathepsin G (HLC-G) were finally eluted with 50 mM acetate, 1.0 M NaCl, 20% DMSO, pH 5.0. The column was developed at 6 ml/min with the collection of 10 ml fractions. The active fractions were pooled, dialyzed vs. two 6 liter changes of 50 mM acetate, 0.1 M NaCl, pH 5.5, and concentrated to 40 ml on an Amicon® ultrafiltration unit (YM-10 membrane). CM-Chromatography: The concentrated active fraction was applied to a column of CM-Sephadex® C-50 (2.2 x 10 cm) previously equilibrated with 50 mM acetate, 0.1 M NaCl, pH 5.5 and the column then washed with this buffer to remove contaminating protein. Elution was continued with 50 mM acetate, 0.2 M NaCl, pH 5.5 and resulted in the displacement of a peak of activity assayed against Bz-L-Phe-L-Val-L-Arg-pNA. HLE was next eluted with the acetate buffer containing 0.45 M NaCl, while elution of HLC-G required the presence of 1.0 M NaCl in the buffer as described by R. Martodam et al. in Preparative Biochemistry, (1979) 9, 15-31. This column was developed at 30 ml/hr with the collection of 5.5 ml fractions. From the thus purified HLE, a standard rate of production of p-nitroaniline was measured at 25°C spectrophotometrically in the visible spectrum at 410 nanometers with automatic data acquisition from a Cary 210 spectrophotometer obtained from Varian Associates. Reactions were initiated by injection of 10 microliters of the HLE solution into a 3 milliliter cuvette containing 2.89 milliliters of buffer (10 millimolar sodium phosphate, 500 millimolar NaCl, pH 7.6), 50 microliters substrate solution in DMSO, and 50 microliters of DMSO. Initial, steady-state reaction velocities of p-nitroaniline production were calculated by a fit of the experimental data to a linear dependence on time by linear least squares. This velocity, determined with no inhibitor present, was used as a standard in the calculation of inhibitor $K_i$ values.

As a general rule, the peptide derivatives of the present invention were found to be "slow-binding" inhibitors of HLE and therefore required special methods of analysis to accurately determine $K_i$ values for their inhibition of HLE (see Williams, J. W. and Morrison, J. F., Meth. Enz. (1979) 63, 437 for a description of these methods.) In a typical experiment, 2.89 ml of buffer (10 millimolar sodium phosphate, 500 millimolar sodium chloride, pH 7.6), 50 microliters of inhibitor solution in DMSO, and 50 microliters of substrate solution in DMSO were added to a 3 milliliter cuvette. The cuvette was stoppered, inverted several times to mix its contents and maintained at (25°C) in the spectrophotometer. After a period of five minutes to allow the reaction solution to come to thermal equilibrium, 10 microliters of stock enzyme solution were added to the cuvette to initiate the reaction. Duplicate or triplicate runs were done at zero inhibitor concentration and at least three non-zero inhibitor concentrations. $K_i$ values were calculated according to methods outlined in the above reference by Williams and Morrison. The $K_i$ values for selected compounds were less than $10^{-7}$M.

## Animal Models

Animal models of emphysema include intratracheal (i.t.) administration of an elastolytic protease to cause a slowly progressive, destructive lesion of the lung. These lesions are normally evaluated a few weeks to a few months after the initial insult. However, these proteases also induce a lesion that is evident in the first few hours. The early lesion is first hemorrhagic, progresses to an inflammatory lesion by the end of the first 24 hours and resolves in the first week post insult. To take advantage of this early lesion, the following model was used.

Hamsters are first lightly anesthetized with methohexital sodium (Brevital® from Eli Lilly). Phosphate buffered saline (PBS) pH 7.4, either alone or containing 400 μg of human leukocyte elastase (HLE), is then administered directly into the trachea. Twenty-four hours later the animals are killed and the lungs removed

and carefully trimmed of extraneous tissue. Following determination of wet lung weight, the lungs are lavaged with PBS and total lavaegable red and white cells recovered are determined. The values for wet lung weights, total lavageable red cells and total lavageable white cells are elevated in a dose-dependent manner following administration of HLE. Compounds that are effective elastase inhibitors can prevent or diminish the severity of the enzyme-induced lesion resulting in lower wet lung weight and reduced values for total lavageable cells, both red and white, relative to administration of HLE alone. Compounds can be evaluated by administering them either with or at various times prior to administration of HLE to determine their utility in preventing an HLE lesion. Compounds of this invention produced statistically significant reductions in wet lung weight and total lavageable cells relative to HLE alone.

Compounds of the present invention exhibited activity in at least one of the tests described above under Inhibition Measurement or Animal Model. It should be noted that there was not always a direct correlation between the activities of the compounds measured as $K_i$ values in the Inhibition Measurement test and the reduced values for total lavageable cells and wet lung weights relative to the administration of HLE alone obtained in the Animal Model test. It is thought that the Animal Model test is more predictive of the activity of such compounds in the treatment of emphysema.

Pharmacokinetics: Male Syrian hamsters (80 to 120g) are injected intravenously with the test compound. Prior to injection and at varying time periods thereafter, they are lightly anesthetized with ether and blood samples of approximately 0.2 ml each are withdrawn by cardiac puncture. The blood is expressed into 2 ml centrifuge tubes and allowed to clot for one hour. The sample is then centrifuged and the serum removed.

Drug levels are determined by first inactivating endogenous elastase inhibitors by incubation of 50 microliters of serum with an equal volume of buffer containing 5 mg/ml bovine pancreatic trypsin for 5 min. The trypsin inactivated serum (10 microliters) is then added to a 0.52 ml cuvette containing buffer made 20 nM with respect to HLE. After an additional 30 min. incubation, the reaction is started by the addition of substrate (350 microliters) (MeOSuc-L-Ala-L-Ala-L-Pro-L-Val-pNA, 1.6 mM) and the reaction monitored spectrophotometrically at a wavelength of 410 nM. For comparative purposes, serum persistence of the test compounds is determined in the following manner:

Percent inhibition of serum samples was calculated as follows:

$$\text{percent inhibition} = \frac{Vo - Vi}{Vo} \times 100$$

The compounds of the present invention may be administered to a warm-blooded animal in need thereof, particularly a human, for the treatment of conditions of pulmonary emphysema, atherosclerosis, rheumatoid arthritis, and osteo arthritis, but in particular for emphysema. The mode of administration may be oral, parenteral, including the subcutaneous deposit by means of an osmotic pump, or via a powdered or liquid aerosol. These may be conventionally formulated in an oral or parenteral dosage form by compounding about 10 to 250 mg per unit of dosage with conventional vehicle, excipient, binder, preservative, stabilizer, flavor or the like as called for by accepted pharmaceutical practice e.g. as described in U.S. Patent No. 3,755,340. For parenteral administration, a 1 to 10 ml intravenous, intramusular or subcutaneous injection would be given containing about 0.02 to 10 mg/kg of body weight of a compound of the invention 3 or 4 times daily. The injection would contain a compound of the invention in an aqueous isotonic sterile solution or suspension optionally with a preservative such as phenol or a solubilizing agent such as ethylenediaminetetraacetic acid (EDTA). In a powdered aerosol, compounds of the invention may be administered in the same manner as cromolyn sodium via a Spinhaler® turbo-inhaler device obtained from Fisons Corp. of Bedford, Massachusetts at a rate of about 0.1 to 50 mg per capsule, 1 to 8 capsules being administered daily for an average human. Each capsule to be used in the Spinhaler® contains the required amount of a compound of the invention with the remainder of the 20 mg capsule being a pharmaceutically-acceptable carrier such as lactose. In a liquid aerosol, the compounds of the invention are administered at the rate of about 100 to 1000 micrograms per "puff" or activated release of a standard volume of propellant. The liquid aerosol would be given at the rate of 1 to 8 puffs per day with variation in dosages due to the severity of the condition being treated, the weight of the patient and the particle size distribution of the aerosol since smaller particles will achieve greater lung penetration. Propellants, e.g., a fluorinated hydrocarbon or isobutane, containers, valves and actuators for liquid aerosols are described by L. Lachman et al. in "The Theory and Practice of Industrial Pharmacy", Lea and Febiger, Philadelphia (1976).

In the following Examples and throughout the specification, the following abbreviations are used: atm (atmospheres) with $1.013 \times 10^5$ Pascals = 1 atm; bp (boiling point); °C (degrees Centigrade); g (grams); hr

(hours); mg (milligrams); min (minutes); ml (milliliters); mmol (millimoles); mp (melting point); N (normal); nm (nanometers); nM (nanomolar); $R_f$ (relative mobility in TLC); TLC (thin layer chromatography); DCC (dicyclohexylcarbodiimide); DMF (dimethylformamide); DMSO (dimethyl sulfoxide); $Et_2O$ (diethyl ether); EtOAc (ethyl acetate); HOAc (acetic acid); WSCDI (water soluble carbodiimide; 1-ethyl-3-(3-dimethylaminopropyl)carbodiimide hydrochloride); Bz (benzoyl); HOBT (1-hydroxybenzotriazole); MeOH (methyl alcohol); Pd/C (palladium on charcoal catalyst); pNA (paranitroanilide); DMPA (4-dimethylaminopyridine); Ø (phenyl group); NMM (N-methylmorpholine); THF (tetrahydrofuran); CBZ (benzyloxycarbonyl); t-BOC (tertiarybutyloxycarbonyl); $t_R$ (HPLC retention time in min); HPLC (high performance liquid chromatography); TEA (triethylamine); TFA (trifluoroacetic acid); $Ac_2O$ (acetic anhydride); RT (room temperature); e.g. (for example); supra (above); DAST (diethylaminosulfurtrifluoride); vs. (versus); Dibal (diisobutylaluminum hydride); and Zorbax® ODS analytical column (4.6 mm x 25 cm) also called "$C_{18}$ column"; Phenomenex Zorbax $C_8$ 25 cm x 4.6 mm, also called "$C_8$ column". In addition, C, H, N, etc. (the conventional symbols for the elements) are used, and conventional abbreviations for amino acids, e.g. proline (Pro), valine (Val) etc. are also used. It is to be understood that generic terms such as "(1-10C)alkyl" include both straight and branched chain alkyl radicals, but references to individual alkyl radicals such as "propyl" include only the straight chain ("normal") radical, branched chain isomers such as "isopropyl" being specifically referred to. $^1H$ NMR data is given for values of delta using tetramethylsilane as an internal standard. Where needed, multiple runs of selected processes were done to obtain additional product.

Flash chromatography was carried out on Merck Kieselgel (Art 9385) and column chromatography on Merck Kieselgel 60 (Art 7734) [these materials were obtained from E. Merck, Darmstadt, W. Germany] or flash and column chromatographies were done on an acidic silica gel (J.T. Baker Chemical Co., low pH, Number 7290-R); thin layer chromatography (TLC) was carried out on Analtech 0.25 mm silica gel GHLF plates (Art 21521), obtainable from Analtech, Newark, DE, USA;

None of the compounds tested have been observed to be toxic.

Reference Example 1

N-[2,2-Difluoro-5-methyl-1,3-dioxo-4-[[(phenylmethoxy)carbonyl]amino]hexyl]-L-phenylalanine methyl ester - (Formula Ia, $R^1 = CH(CH_3)_2$, $R^3$A-Ø$CH_2OCO$-, $R^B$ = Formula IIb $R^6 = CH_2$Ø, $R^8 = OCH_3$, $R^C = H$)

a. N-Benzyloxycarbonyl-L-valinol (Formula XIVa, $R^3A$ = Ø$CH_2OCO$-, $R^1 = CH(CH_3)_2$)

Benzyl chloroformate (91.0 g, 0.532 mol, 95% purity) was added dropwise over a period of 1 hr to a pre-cooled (0 °C) solution of L-valinol (50.0 g, 0.484 mol) and triethylamine (60.0 g, 0.6 mol) in $CHCl_3$ (1500 ml). The reaction mixture was stirred for 1 hr. at 0 °C and then allowed to warm to room temperature over 2 hr. The reaction mixture was concentrated under vacuum. EtOAc (1500 ml) was added to the resulting residue and the organic solution was washed with aqueous 1N NaOH and brine. The organic phase was dried over $MgSO_4$, filtered and concentrated under vacuum. The resulting residue was purified by flash chromatography on a column of silica gel (6cm x 30cm) using a stepwise gradient of $Et_2O$:hexane (1:5) followed by pure $Et_2O$ to give the product (91.4 g) as a white waxy solid; TLC, $R_f = 0.23$, silica gel, hexane:$Et_2O$ (50:50).

b. N-Benzyloxycarbonyl-L-valinal (Formula VIa, $R^3A$ = Ø$CH_2OCO$-, $R^1 = CH(CH_3)_2$)

A solution of DMSO (107.2 g, 1.372 mol) in $CH_2Cl_2$ (150 ml) was added dropwise over 0.5 hr to a pre-cooled (-60 °C), stirred solution of oxalyl chloride (87.1 g, 0.686 mol) in $CH_2Cl_2$ (800 ml) under a nitrogen atmosphere. The temperature of the mixture rose to -45 °C. The reaction mixture was then warmed to -30 °C. A solution of the product of Example 1a (81.5 g, 0.343 mol) in $CH_2Cl_2$ (300 ml) was added dropwise over 45 min at -30 °C. The reaction mixture was stirred for 50 min at -25 °C, cooled to -40 °C and a solution of diisopropylethyl amine (177.4 g, 1.372 mol) in $CH_2Cl_2$ (250 ml) was added dropwise over 45 min at -40 °C. The reaction mixture was stirred for 1 hr as it warmed to room temperature. The reaction mixture was diluted with $CH_2Cl_2$ (1500 ml) and the organic phase was washed with aqueous 1N HCl and then concentrated under vacuum to give the product (98 g) as a green oil which was used immediately without further purification; TLC, $R_f = 0.48$, silica gel, hexane:$Et_2O$ (50:50).

c. N-Benzyloxycarbonyl-L-valinal diethylacetal (Formula XVa, $R^3A = ØCH_2OCO$-, $R^1 = CH(CH_3)_2$)

Triethyl orthoformate (700 g, 4.723 mol), absolute EtOH (800 ml) and p-toluenesulfonic acid monohydrate (5.0 g, 0.026 mol) were added to the product of Example 1b (81 g, 0.343 mol). The mixture was stirred for 10 min at room temperature and then concentrated under vacuum. The resulting residue was dissolved in $Et_2O$ and washed with saturated aqueous $NaHCO_3$. The organic phase was dried over $Na_2SO_4$, filtered and concentrated under vacuum to give a crude product. This product was purified by flash chromatography with silica gel using a stepwise gradient of hexane through mixtures of $CH_2Cl_2$:hexane to EtOAc: $CH_2Cl_2$ (30:70) to give the product as a pale yellow oil; TLC, $R_f = 0.21$, silica gel, $CH_2Cl_2$:petroleum ether (50:50).

d. L-Valinal diethylacetal (Formula XVIIIa, $R^1 = CH(CH_3)_2$)

A mixture of a product made by the method of Example 1c (147.8 g, 0.478 mol) and 10% Pd/C (10 g) in EtOAc (1500 ml) was stirred under $H_2$ (1 atm) until 2500 ml of $H_2$ was consumed. Twice during this time the reaction was interrupted and 10% Pd/C (10 g) was added. The reaction mixture was then filtered through a pad of diatomaceous earth. 10% Pd/C (10 g) was added and the reaction mixture stirred until 10.92 liters of $H_2$ was consumed. The reaction mixture was filtered through diatomaceous earth and the filtrate was concentrated under vacuum to give the product (78.8 g) as a pale yellow oil; $[alpha]_D25 = +7.8$.

e. (Formula VIIa, $R^3A = ØCH_2OCO$-, $R^1 = CH(CH_3)_2$)

The compound of Example 1b (3.0 g, 12.7 mmol), ethyl 2-bromo-2,2-difluoroacetate (2.58 g, 12.7 mmol) and Zn dust (1.22 g) were refluxed in THF (30 ml) for 0.5 hr under $N_2$. Additional Zn dust (1.22 g) and ethyl 2-bromo-2,2-difluoroacetate (2.58 g, 12.7 mmol) were added and the resulting solution refluxed for an additional hour. The solution was cooled to room temperature and ethyl acetate (150 ml) was added. The ethyl acetate solution was washed with 1M $KHSO_4$ and brine, dried ($Na_2SO_4$), filtered and the solvent removed under reduced pressure to give a crude product (2.4 g). The product was purified by flash chromatography on silica gel with an eluent of ethyl acetate/hexane (35:65) to give the final product (0.9 g) as an oil; TLC, $R_f = 0.55$, ethyl acetate:hexane (35:65).

f. (Formula VIIIa, $R^1 = CH(CH_3)_2$, $R^3A = ØCH_2OCO$-, $R^B$ = Formula IIb, $R^6 = CH_2Ø$, $R^8 = OCH_3$, $R^C = H$)

Tetramethylguanidine (TMG) (0.255 g, 2.2 mmol) was added to a stirred solution of the product from 1e, L-phenylalanine methyl ester hydrochloride (0.16 g, 0.74 mmol), and chloroform (4 ml) chilled in an ice bath under $N_2$. The ice bath was removed and the reaction was allowed to come to room temperature and then stirred for 48 hours. $CHCl_3$ (25 ml) was added to the reaction mixture and the resulting solution was washed with 5% $NaHCO_3$ and brine, dried ($MgSO_4$), filtered and the filtrate was concentrated under vacuum to afford a crude product (0.69 g). The product was purified by flash chromatography (using $CHCl_3$, then $CHCl_3$:EtOAc (98:2)), to give the product (0.15 g, 42%) as a viscous gum; TLC, $R_f = 0.46$, $CHCl_3$: EtOAc (8:2).

g. (Formula Ia, $R^1 = CH(CH_3)_2$, $R^3A = ØCH_2OCO$-, $R^B$ = Formula IIb, $R^6 = CH_2Ø$, $R^8 = OCH_3$, $R^C = H$)

To a solution of the product of Example 1f (0.15 g, 0.304 mmol) and dry $CH_2Cl_2$ (5.0 ml) was added Dess-Martin periodinane (1.29 g, 3.04 mmol). Trifluoroacetic acid (0.4 ml) was added and the solution was allowed to stir at room temperature under $N_2$ overnight. EtOAc (10 ml) was added and the mixture was extracted with saturated $Na_2S_2O_3$, saturated $NaHCO_3$ and brine. The organic layer was dried ($MgSO_4$) and concentrated under vacuum to afford, after purification by flash chromatography, (using $CHCl_3$, then MeOH:$CHCl_3$ (2:98)), the product (0.04 g) as an oil; TLC, $R_f = 0.9$, MeOH:$CHCl_3$ (2:98).

| Analysis calculated for | | | |
|---|---|---|---|
| $C_{25}H_{28}F_2N_2O_6.0.5\ H_2O$: | C, 60.15; | H, 5.85; | N, 5.61 |
| Found: | C, 60.42; | H, 5.69; | N, 5.29 |

Example 2

2-[[[4-[(1-Aminocarbonyl-2-phenylethyl)amino]-3,3-difluoro-1-(1-methylethyl)-2,4-dioxobutyl]amino]carbonyl]-1-pyrrolidinecarboxylic acid phenylmethyl ester (Formula Ib, $R^1 = CH(CH_3)_2$, $R^3A = ØCH_2OCO$-, $R^B$ = Formula IIb, $R^6 = CH_2Ø$, $R^8 = NH_2$, $R^C = H$)

a. (Formula XVb, $R^1 = CH(CH_3)_2$, $R^3A = ØCH_2OCO$-)

WSCDI (24.04 g, 125.4 mmol) was added to a stirred solution of CBZ-L-proline (28.4 g, 114.1 mmol), HOBT (30.00 g, 228.2 mmol), the product of Example 1d (20 g, 114.1 mmol) and dry THF (400 ml) at 0 °C under $N_2$. The resulting mixture was stirred at 0 °C for 1 hr; the cooling bath was removed and the mixture was allowed to warm to room temperature and was stirred at room temperature overnight. The THF was removed under vacuum to afford the crude product. The crude product was dissolved in ethyl acetate and the ethyl acetate solution was successively washed with 1N HCl, saturated $NaHCO_3$, and brine. The ethyl acetate solution was then dried ($MgSO_4$), filtered and the filtrate was concentrated under vacuum to afford, after purification by flash chromatography ($CHCl_3$:MeOH (98:2)), the product (35.03 g, 77%) as a yellow oil; TLC, $R_f = 0.7$, $CHCl_3$:MeOH (95:5).

b. (Formula VIb, $R^1 = CH(CH_3)_2$, $R^3A = ØCH_2OCO$-)

A solution of the product from Example 2a (24.0 g, 60 mmol), p-toluenesulfonic acid (2.4 g, 12.6 mmol) and acetone (1600 ml) was stirred at room temperature for 5 hr. The acetone was removed under vacuum to leave an oily residue. This residue was dissolved in chloroform and the solution was washed with saturated $NaHCO_3$ and brine, dried ($MgSO_4$), filtered and the filtrate was concentrated under vacuum to give the product (17.67 g, 91%) as a light amber oil; TLC, $R_f = 0.46$, $CHCl_3$:$CH_3OH$ (95:5).

c. (Formula VIIb, $R^1 = CH(CH_3)_2$, $R^3A = ØCH_2OCO$-)

A mixture of the product from Example 2b (2.50 g, 7.75 mmol), ethyl 2-bromo-2,2-difluoroacetate (1.57 g, 7.75 mmol), activated zinc (0.505 g, 7.75 mmol) and dry THF (125 ml) was heated at gentle reflux under nitrogen for 1 hr. The mixture was then allowed to cool to just below reflux and additional ethyl 2-bromo-2,2-difluoroacetate (1.57 g, 7.75 mmol) and activated zinc (0.505 g, 7.75 mmol) were added. The reaction mixture was again heated to gentle reflux and kept at reflux for 3 hr. The mixture was cooled and ethyl acetate (400 ml) was added. The ethyl acetate solution was washed with 1N $KHSO_4$ solution and brine, dried over $MgSO_4$, filtered and the filtrate was concentrated under vacuum to afford a crude product (3.7 g). The product was purified by flash chromatography (EtOAc:hexane (1:1)) to give 1.53 g (45% yield) of the desired product as a light yellow waxy solid; TLC, $R_f = 0.45$, petroleum ether:EtOAc (33:66).

d. (Formula IXb, $R^1 = CH(CH_3)_2$, $R^3A = ØCH_2OCO$-)

1N Sodium hydroxide solution (2.75 ml, 2.75 mmol) was added to a stirred solution of the product from Example 2c (1.0 g, 2.19 mmol) and methanol (15 ml) at room temperature. The resulting solution was stirred at room temperature for 4 hr. The reaction mixture was treated with water (75 ml) and the resulting solution was extracted with EtOAc. The aqueous layer was made acidic (pH 2) with 1N HCl. The acidic aqueous mixture was extracted with ethyl acetate, and the ethyl acetate layer was washed with brine, dried ($MgSO_4$), filtered and the filtrate was concentrated under vacuum to give the product (0.883 g, 94.1% yield) as a white foam; TLC, $R_f = 0.1$, $CHCl_3$: MeOH:HOAc (95:5:0.5).

e. (Formula VIIIb, $R^1 = CH(CH_3)_2$, $R^3A = ØCH_2OCO$-, $R^B$ = Formula IIb, $R^6 = CH_2Ø$, $R^8 = NH_2$, $R^C = H$)

WSCDI was added to a stirred solution of the product from Example 2d (0.25 g, 0.583 mmol), L-phenylalanine amide (0.10 g, 0.609 mmol), HOBT (0.087 g, 0.64 mmol), and dry THF (10 ml) at 0 °C under $N_2$. The resulting mixture was stirred at 0 °C for 1 hr; then the cooling source was removed and the reaction mixture was allowed to warm to room temperature and stir at room temperature overnight. The mixture was concentrated under vacuum, and the resulting residue was dissolved in ethyl acetate. The ethyl acetate solution was washed, successively, with 1N HCl, saturated $NaHCO_3$ and brine, dried ($MgSO_4$), filtered and the filtrate was concentrated under vacuum to afford, after purification by flash chromatography ($CHCl_3$:MeOH (95:5)), the product (72%); TLC, $R_f = 0.58$, $CHCl_3$:$CH_3OH$ (90:10).

f. (Formula Ib, $R^1 = CH(CH_3)_2$, $R^3A = ØCH_2OCO$-, $R^B =$ Formula IIb, $R^6 = CH_2Ø$, $R^8 = NH2$, $R^C = H$)

Acetic anhydride (3 ml, 27.2 mmol) was added to a stirred solution of the product from Example 2e (0.23 g, 0.4 mmol) and dry DMSO (3 ml, 34.8 mmol) at room temperature under $N_2$. The resulting mixture was stirred overnight at room temperature. The mixture was then poured onto ice/water (40 ml) and stirred vigorously for 3 hr. The aqueous mixture was extracted (2 times) with ethyl acetate. The ethyl acetate layer was washed (2 times) with saturated $NaHCO_3$ and brine, dried ($MgSO_4$), filtered and the filtrate concentrated under water vacuum to afford, after purification by flash chromatography ($CHCl_3$:MeOH (95:5)) the product (0.18 g, 79%); TLC, $R_f = 0.68$, $CHCl_3:CH_3OH$ (90:10).

| Analysis calculated for | | | |
|---|---|---|---|
| $C_{29}H_{34}F_2N_4O_6.1.0\ H_2O$: | C, 58.97; | H, 9.14; | N, 9.49 |
| Found: | C, 59.07; | H, 6.03; | N, 9.15 |

Example 3

N-[2,2-Difluoro-5-methyl-1,3-dioxo-4-[[[1-[(phenylmethoxy)carbonyl]-2-pyrrolidinyl]carbonyl]amino]-hexyl]-L-seryl-L-phenylalaninamide (Formula Ib, $R^1 = CH(CH_3)_2$, $R^3A = ØCH_2OCO$-, $R^B =$ Formula IIa, $R^6 = CH_2OH$, $R^7 = CH_2Ø$, $R^8 = NH_2$, $R^C = H$)

a. (Formula XXVIb, $R^1 = CH(CH_3)_2$, $R^3A = ØCH_2OCO$-)

A solution of the product of Example 2c (1.3 g, 2.85 mmol) and $CH_2Cl_2$ (5.0 ml) was added to a stirred mixture of Dess-Martin periodinane (4.83 g, 11.39 mmol) and $CH_2Cl_2$ (35 ml). Trifluoroacetic acid (0.89 ml) was added and the reaction mixture was stirred at room temperature under $N_2$ overnight. Ethyl acetate (200 ml) was added and the mixture was extracted with saturated $Na_2S_2O_3$, saturated $NaHCO_3$ and brine. The organic layer was dried ($MgSO_4$), filtered and concentrated under vacuum to afford, after purification by flash chromatography (MeOH:$CHCl_3$ (1:99)), the product (0.53 g); TLC, $R_f = 0.65$, MeOH: $CHCl_3$ (2:98).

b. (Formula XXVIIb, $R^1 = CH(CH_3)_2$, $R^3A = ØCH_2OCO$-)

To a solution of the product of Example 3a (2.0 g, 4.4 mmol) and MeOH (20 ml) and was added 1N NaOH (6.16 ml, 6.16 mmol). The solution was stirred at room temperature for 3 hr. The MeOH was removed under vacuum and $H_2O$ (75 ml) was added to the residue. The aqueous solution was extracted with EtOAc and the EtOAc layer was discarded. The aqueous solution was taken to pH 2.0 with 1N HCl and extracted 3 times with EtOAc. The combined organic layers were washed with brine, dried ($MgSO_4$), filtered and concentrated under vacuum to give the product (1.51 g, 80.5%) as a white foam; NMR (DMSO-$d_6$): 0.82 (m,6), 1.8 (m,3), 2.17 (m,2), 3.4 (m,2), 4.36 (m,1), 4.7 (m,1), 4.95 (m,2), 7.3 (m,5), 8.37 (m,1).

c. (Formula Ib, $R^1 = CH(CH_3)_2$, $R^3A = ØCH_2OCO$-, $R^B =$ Formula IIa, $R^6 = CH_2OH$, $R^7 = CH_2Ø$, $R^8 = NH_2$, $R^C = H$)

Using the method of Example 2e, the product from Example 3b was allowed to react with L-serine-L-phenylalanineamide and one equivalent of N-methylmorpholine to afford, after purification by flash chromatography (MeOH:$CHCl_3$ (5:95)), the product (44%); TLC, $R_f = 0.50$, MeOH:$CHCl_3$ (10:90).

| Analysis calculated for | | | |
|---|---|---|---|
| $C_{32}H_{39}F_2N_5O_8.0.75H_2O$: | C, 57.09; | H, 6.06; | N, 10.40 |
| Found: | C, 57.14; | H, 5.96; | N, 10.07 |

Example 4

2-[[[3,3-Difluoro-4-[(1-methoxycarbonyl-2-phenylethyl)amino]-1-(1-methylethyl)-2,4-dioxobutyl]amino]-carbonyl]-1-pyrrolidinecarboxylic acid phenylmethyl ester (Formula Ib, $R^1 = CH(CH_3)_2$, $R^3A = ØCH_2OCO$-, $R^B$ = Formula IIb, $R^6 = CH_2Ø$, $R^8 = OCH_3$, $R^C = H$)

Using the method of Example 2e, product prepared using the method of Example 3b was allowed to react with L-phenylalanine methyl ester hydrochloride and one equivalent of N-methylmorpholine. The reaction afforded, after purification by flash chromatography ($MeOH:CHCl_3$ (5:95)), the title product (62%); TLC, $R_f = 0.65$, $MeOH:CHCl_3$ (5:95).

| Analysis calculated for | | | |
|---|---|---|---|
| $C_{30}H_{35}F_2N_3O_7$: | C, 61.32; | H, 6.00; | N, 7.15 |
| Found: | C, 61.25; | H, 6.08; | N, 7.15 |

Example 5

2-[[[4-[(1-Carboxy-2-phenylethyl)amino]-3,3-difluoro-1-(methylethyl)-2,4-dioxobutyl]amino]carbonyl]-1-pyrrolidinecarboxylic acid phenylmethyl ester (Formula Ib, $R^1 = CH(CH_3)_2$, $R^3A = ØCH_2OCO$-, $R^B$ = Formula IIb, $R^6 = CH_2Ø$, $R^8 = OH$, $R^C = H$)

Using the method of Example 3b, product prepared according to the method of Example 4 was allowed to react with 1N NaOH to afford the product (67%); HPLC, $t_R = 7.92$ min, $CH_3CN:H_2O$ (45:55), 2.0 ml/min, $C_{18}$ column.

| Analysis calculated for | | | |
|---|---|---|---|
| $C_{29}H_{33}F_2N_3O_7 . H_2O$: | C, 58.87; | H, 5.96; | N, 7.10 |
| Found: | C, 58.63; | H, 5.68; | N, 6.90 |

Example 6

(No example for this number.)

Example 7

2-[[[3,3-difluoro-4-[(methoxycarbonylmethyl)amino]-1-(1-methylethyl-2,4-dioxobutyl]amino]carbonyl]-1-pyrrolidinecarboxylic acid phenylmethyl ester (Formula Ib, $R^3A = ØCH_2OCO$-, $R^1 = CH(CH_3)_2$, $R^B$ = formula IIb, $R^6 = H$, $R^8 = -OCH_3$, $R^C = H$)

a. (Formula VIIIa, $R^3A = ØCH_2OCO$-, $R^1 = CH(CH_3)_2$, $R^B$ = formula IIb, $R^6 = H$, $R^8 = OCH_3$, $R^C = H$)

Using the method of Example 2e, a product prepared by the method of Example 2d was allowed to react with glycine methyl ester hydrochloride and 1 equivalent of N-methylmorpholine to afford a product (86%); TLC, $R_f$0.38, $MeOH:CHCl_3$ (5:95), silica gel.

b. (Formula Ib, $R^3A = ØOCO$-, $R^1 = CH(CH_3)_2$, $R^B$ = formula IIb, $R^6 = H$, $R^8 = OCH_3$, $R^C = H$)

To a solution of the product of Example 7a (0.97 g, 1.94 mmoles) and dry $CH_2Cl_2$ (25 ml) was added Dess-Martin periodinane (4.13 g, 9.71 mmoles). Trifluoroacetic acid (0.76 ml) was added and the solution was allowed to stir at room temparature under $N_2$ overnight. EtOAc (50 ml) was added and the mixture was poured into saturated $NaHCO_3$ containing $Na_2S_2O_3$ (8 g, 32 mmoles) and stirred vigorously for 2 minutes. The EtOAc layer was separated and extracted with saturated $NaHCO_3$ and brine. The EtOAc solution was concentrated under vacuum to afford, after purification by flash chromatography ($MeOH:CHCl_3$, 2:98), a

product (55%); TLC, $R_f = 0.65$, silica, $CHCl_3$:MeOH (95:5).

| Analysis calculated for: | | | |
|---|---|---|---|
| $C_{23}H_{29}F_3N_3O_7.0.25\ H_2O$: | C, 55.03; | H, 5.92; | N, 8.37 |
| Found: | C, 55.09; | H, 5.84; | N, 8.21 |

Example 8

2-[[[4-[(carboxymethyl)amino]-3,3-difluoro-1-(1-methylethyl)-2,4-dioxobutyl]amino]carbonyl]-1-pyrrolidinecarboxylic acid phenylmethyl ester (Formula Ib, $R^3A = ØCH_2OCO$-, $R^1 = CH(CH_3)_2$, $R^B$ = formula IIb, $R^6$ = H, $R^8$ = OH, $R^C$ = H)

Using the method of Example 3b, but using $H_2O$:MeOH in a ratio of 1:4 and allowing the reaction to stir overnight, the product of Example 7b was allowed to react with 1N NaOH to afford, after purifying by flash chromatography on acidic silica gel (MeOH:$CHCl_3$, (5:95)), a product (44%); TLC, $R_f$ = -0.25 and 0.32 ($CH_3OH$: $CHCl_3$:HOAc, 5:95:0.1).

| Analysis calculated for | | | |
|---|---|---|---|
| $C_{22}H_{27}F_2N_3O_7.0.5\ H_2O$: | C, 53.66; | H, 5.73; | N, 8.53 |
| Found: | C, 53.55; | H, 5.53; | N, 8.15 |

Example 9

N-[N-[2,2-difluoro-5-methyl-1,3-dioxo-4-[[[1-[(phenylmethoxy)carbonyl]-2-pyrrolidinyl]carbonyl]amino]hexyl]-L-phenylalanyl]glycine methyl ester (Formula Ib, $R^3A = ØCH_2OCO$-, $R^1 = CH(CH_3)_2$, $R^B$ = formula IIa, $R^6 = CH_2Ø$-, $R^7$ = H, $R^8 = OCH_3$, $R^C$ = H)

a. (N-Benzyloxycarbonyl-L-phenylalanylglycine methyl ester)

Using the method of Example 7a, CBZ-L-phenylalanine was allowed to react with glycine methyl ester hydrochloride to afford, after purification by flash chromatography ($CHCl_3$), a product (61%); TLC, $R_f = 0.69$, MeOH:$CHCl_3$ (5:95), silica gel.

b. (L-Phenylalanylglycine methyl ester)

The compound of Example 9a (2.2 g, 5.94 mmoles) was placed in absolute EtOH (250 ml) and warmed to effect solution. After cooing to room temperature, 10% Pd/C was added and the mixture was hydrogenated on a Parr shaker at 3 atmospheres of $H_2$ for 3 hours. The mixture was filtered through diatomaceous earth (Celite). The filtrate was concentrated under reduced pressure to give a product (1.32 g, 94%) as a pale yellow oil. The product was used without further purification.

c. (Formula VIIIb, $R^3A = ØCH_2OCO$-, $R^1 = CH(CH_3)_2$, $R^B$ = formula IIa, $R^6 = CH_2Ø$, $R^7$ = H , $R^8 = OCH_3$, $R^C$ = H)

Using the method of Example 2e, a product prepared by the method of Example 2d was allowed to react with the product of Example 9b to afford, without further purification, a product (84%); TLC; $R_f = 0.55$, MeOH:$CHCl_3$ (5:95), silica gel.

d. (Formula Ib, $R^3A = ØCH_2OCO$-, $R^1 = CH(CH_3)_2$, $R^B$ = formula IIa, $R^6 = CH_2Ø$ , $R^7$ = H, $R^8 = OCH_3$, $R^C$ = H)

Using the method of Example 7b, the product of Example 9c was oxidized to afford, after purification by flash chromatography ($CHCl_3$, $CH_3OH$:$CHCl_3$ (3:97)), the title product (48%); TLC, $R_f = 0.62$, MeOH:$CHCl_3$ - (5:95), silica.

| Analysis calculated for | | | |
|---|---|---|---|
| $C_{32}H_{38}F_2N_4O_8.0.5\ H_2O$:<br>Found: | C, 58.79;<br>C, 58.65; | H, 6.01;<br>H, 5.89; | N, 8.37<br>N, 8.41 |

Example 10

N-[N-[2,2-difluoro-5-methyl-1,3-dioxo-4-[[[1-[(phenylmethoxy)carbonyl]-2-pyrrolidinyl]carbonyl]amino]hexyl]-L-phenylalanyl]glycine (Formula Ib, $R^3A = ØCH_2OCO$-, $R^1 = CH(CH_3)_2$, $R^B$ = formula IIa, $R^6 = CH_2Ø$, $R^7 = H$, $R^8 = OH$, $R^C = H$)

Using the method of Example 8a, the product of Example 9d was allowed to react with 1N NaOH for 2 hours to afford, after purificaton by flash chromatography on acidic silica gel ($CHCl_3$, MeOH:$CHCl_3$ (3:97)), the title product (61%); TLC, $R_f = 0.48$, silica gel, $CHCl_3$:MeOH:HOAc (95:5:0.1).

| Analysis calculated for | | | |
|---|---|---|---|
| $C_{31}H_{36}F_2N_4O_8.\ H_2O$:<br>Found: | C, 57.40;<br>C, 57.29; | H, 5.90;<br>H, 5.74; | N, 8.63<br>N, 8.36 |

Example 11

$N^2$-[2,2-difluoro-5-methyl-1,3-dioxo-4-[[[1-[(phenylmethoxy)carbonyl]-2-pyrrolidinyl]carbonylamino]hexyl]-$N^6$-[(phenylmethoxy)carbonyl]-L-lysine phenylmethyl ester (Formula Ib, $R^3A = ØCH_2OCO$-, $R^1 = CH(CH_3)_2$, $R^B$ = formula IIb, $R^6 = ØCH_2OCONH(CH_2)_4$-, $R^8 = OCH_2Ø$, $R^C = H$)

a. (Formula VIIIb, $R^3A = ØCH_2OCO$-, $R^1 = CH(CH_3)_2$, $R^B$ = formula IIb, $R^6 = ØCH_2OCONH(CH_2)_4$-, $R^8 = OCH_2Ø$, $R^C = H$)

Using the method of Example 7a, a product prepared by the method of Example 2d was allowed to react with $\epsilon$-CBZ-L-lysine benzyl ester hydrochloride to afford, without further purification, a product (88%); TLC, $R_f = 0.68$, silica gel, MeOH:$CHCl_3$ (95:5).

b. (Formula Ib, $R^3A = ØCH_2OCO$-, $R^1 = CH(CH_3)_2$, $R^B$ = formula IIb, $R^6 = ØCH_2OCONH(CH_2)_4$-, $R^8 = OCH_2Ø$, $R^C = H$)

Using the method of Example 7b, the product of Example 11a was oxidized to afford, after purification by flash chromatography (MeOH:$CHCl_3$ (2:98)), the title product (62%); HPLC, $t_R = 8.79$ min, 2 ml/min, $H_2O$:$CH_3CN$:THF:TFA (55:35:15:0.1), $C_8$ column.

| Analysis calculated for | | | |
|---|---|---|---|
| $C_{41}H_{48}F_2N_4O_9.0.5\ H_2O$:<br>Found: | C, 62.50;<br>C, 62.47; | H, 6.14;<br>H, 6.15; | N, 7.11<br>N, 7.08 |

Example 12

$N^2$-[2,2-difluoro-5-methyl-1,3-dioxo-4-[[[1-[(phenylmethoxy)carbonyl)-2-pyrrolidinyl]carbonyl]amino]-hexyl]-$N^6$-[(phenylmethoxy)carbonyl]-L-lysine (Formula Ib, $R^3A = ØCH_2OCO$-, $R^1 = CH(CH_3)_2$, $R^B$ = formula IIb, $R^6 = ØCH_2OCONH(CH_2)_4$-, $R^8 = OH$, $R^C = H$)

Using the method of Example 8a, the product of Example 11b was allowed to react with 1N NaOH for 18 hours to afford, after purificaton by flash chromatography on acidic silica gel (MeOH:$CHCl_3$ (2:98)), the title product (55%); TLC, $R_f = 0.41$, silica gel, MeOH:$CHCl_3$:HOAc (5:95:0.1).

| Analysis calculated for | | | |
|---|---|---|---|
| $C_{34}H_{42}F_2N_4O_9$. $H_2O$: Found: | C, 57.78; C, 57.97; | H, 6.27; H, 6.03; | N, 7.92 N, 7.85 |

Example 13

N-[2,2-difluoro-5-methyl-1,3-dioxo-4-[[[1-[(phenylmethoxy)carbonyl]-2-pyrrolidinyl]carbonyl]amino]hexyl]-L-glutamic acid 1-methyl 5-(phenylmethyl) ester (Formula Ib, $R^3A = ØCH_2OCO$-, $R^1 = CH(CH_3)_2$, $R^B$ = formula IIb, $R^6 = ØCH_2OCONH(CH_2)_2$-, $R^8 = OCH_2Ø$, $R^C = H$)

a. (Formula VIIIb, $R^3A = ØCH_2OCO$-, $R^1 = CH(CH_3)_2$, $R^B$ = formula IIb, $R^6 = ØCH_2OCONH(CH_2)_2$-, $R^8 = OCH_2Ø$, $R^C = H$)

Using the method of Example 7a, a product prepared by the method of Example 2d was allowed to react with L-α,γ-dibenzylglutamate tosylate to afford, without further purification, a product (52%); TLC; $R_f = 0.6$, silica gel, MeOH:$CHCl_3$ (5:95).

b. (Formula Ib, $R^3A = ØCH_2OCO$-, $R^1 = CH(CH_3)_2$, $R^B$ = formula IIb, $R^6 = ØCH_2OCONH(CH_2)_2$-, $R^8 = OCH_2Ø$, $R^C = H$)

Using the method of Example 7b, the product of Example 13a was oxidized to afford, after purification by flash chromatogrpahy ($CHCl_3$), the title product (57%); TLC, $R_f = 0.74$, silica gel, MeOH:$CHCl_3$ (5:95).

| Analysis calculated for | | | |
|---|---|---|---|
| $C_{39}H_{43}F_2N_3O_9$.1.75 $H_2O$: Found: | C, 61.05; C, 61.04; | H, 6.10; H, 5.67; | N, 5.47 N, 5.51 |

Example 14

2-[[[3,3-difluoro-1-(1-methylethyl)-2,4-dioxo-4-[[2-phenyl-1-[(phenylmethoxy)carbonyl]ethyl]amino]butyl]-amino]-carbonyl]-1-pyrrolidinecarboxylic acid phenylmethyl ester (Formula Ib, $R^3A = ØCH_2OCO$-, $R^1$ = CH-$(CH_3)_2$, $R^B$ = formula IIb, $R^6 = CH_2Ø$, $R^8 = OCH_2Ø$, $R^C = H$)

a. (Formula VIIIb, $R^3A = ØCH_2OCO$-, $R^1 = CH(CH_3)_2$, $R^B$ = formula IIb, $R^6 = CH_2Ø$, $R^8 = OCH_2Ø$, $R^C = H$)

Using the method of Example 7a, a product prepared by the method of Example 2d was allowed to react with L-phenylalanine benzyl ester tosylate to afford, without further purification, a product (84%); TLC, $R_f = 0.52$ and 0.65, silica gel, MeOH:$CHCl_3$ (5:95).

b. (Formula Ib, $R^3A = ØCH_2OCO$-, $R^1 = CH(CH_3)_2$, $R^B$ = formula IIb, $R^6 = CH_2Ø$, $R^8 = OCH_2Ø$, $R^C = H$)

Using the method of Example 7b the product of Example 14a was oxidized to afford, after purification by flash chromatography ($CHCl_3$) the title product (29%); TLC, $R_f = 0.81$, silica gel, MeOH:$CHCl_3$ (5:95).

| Analysis calculated for | | | |
|---|---|---|---|
| $C_{36}N_{40}F_2N_3O_7$.0.5 $H_2O$: Found: | C, 64.18; C, 64.23; | H, 6.13; H, 5.88; | N, 6.24 N, 6.14 |

Example 15

2-[[[3,3-difluoro-1-(1-methylethyl)-2,4-dioxo-4-[[[(phenyl-methoxy)carbonylmethyl]amino]butyl]amino]-carbonyl]-1-pyrrolidinecarboxylic acid phenylmethyl ester (Formula Ib, $R^3A = ØCH_2OCO$-, $R^1 = CH(CH_3)_2$, $R^B$ = formula IIb, $R^6 = H$, $R^8 = OCH_2Ø$, $R^C = H$)

a. (Formula VIIIb, $R^3A = ØCH_2OCO$-, $R^1 = CH(CH_3)_2$, $R^B$ = formula IIb, $R^6 = H$, $R^8 = OCH_2Ø$, $R^C = H$)

Using the method of Example 7a a product prepared by the method of Example 2d was allowed to react with glycine benzyl ester tosylate to afford, without further purification, a product (84%); TLC, $R_f = 0.58$, silica gel, MeOH:$CHCl_3$ (5:95).

b. (Formula Ib, $R^3A = ØCH_2OCO$-, $R^1 = CH(CH_3)_2$, $R^B$ = formula IIb, $R^6 = H$, $R^8 = OCH_2Ø$, $R^C = H$)

Using the method of Example 1g, but using 5 equivalents of Dess-Martin Periodinane reagent, the product of Example 15a was oxidized to afford, after purificatoin by flash chromatography ($CHCl_3$) the title product (76%); TLC, $R_f = 0.67$, silica gel, MeOH:$CHCl_3$ (3:97).

| Analysis calculated for | | | |
|---|---|---|---|
| $C_{29}H_{33}F_2N_3O_7 \cdot 0.5\ H_2O$: | C, 59.78; | H, 5.88; | N, 7.21 |
| Found: | C, 59.73; | H, 5.64; | N, 7.19 |

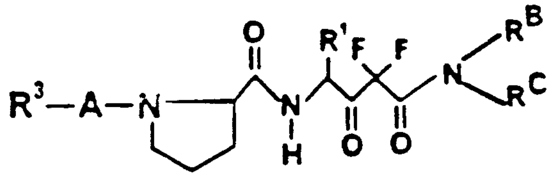

Ib

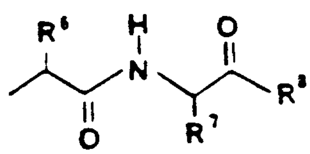

IIa

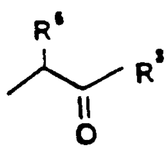

IIb

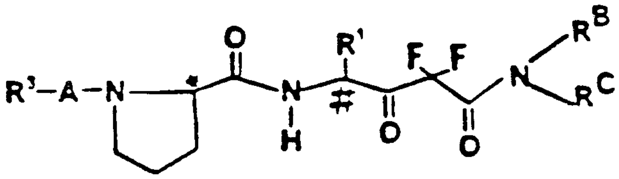

IIIb

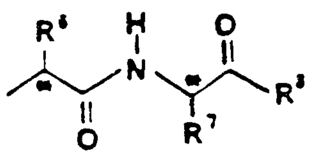

IIId

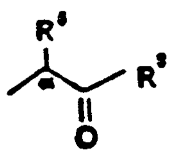

IIIe

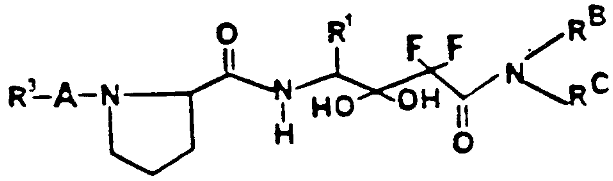

IVb

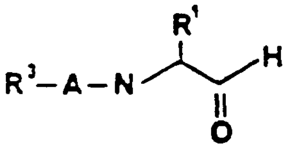

VIa

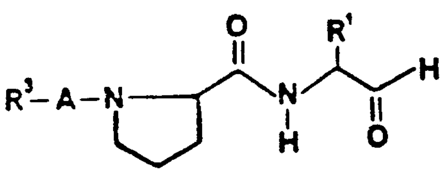

VIb

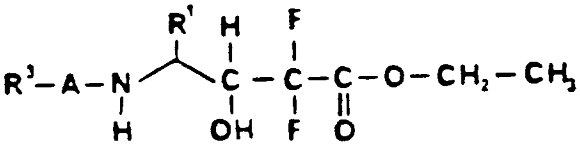

VIIa

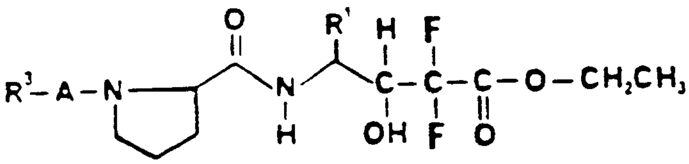

VIIb

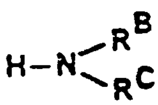

V

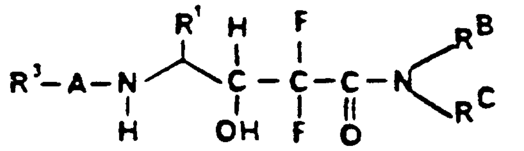

VIIIa

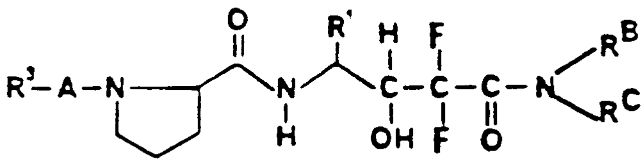

VIIIb

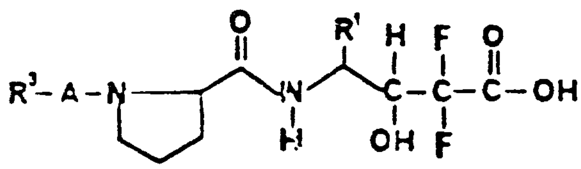

IXb

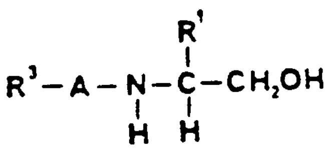

XIVa

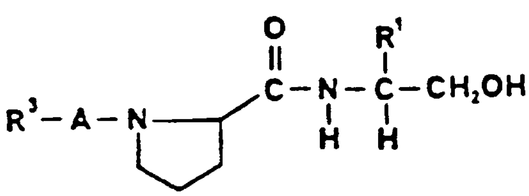

XIVb

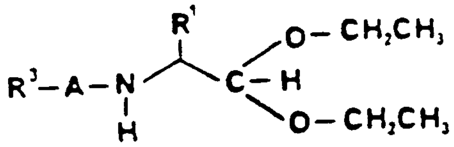

XVa

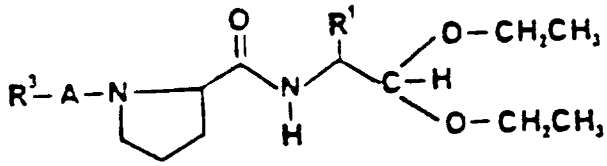

XVb

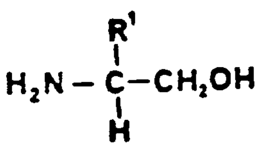

XVI

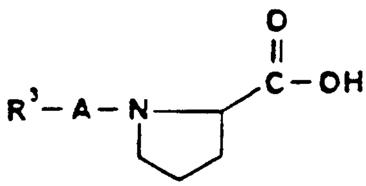

XVIIb

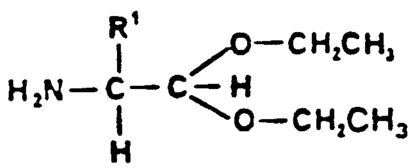

XVIIIa

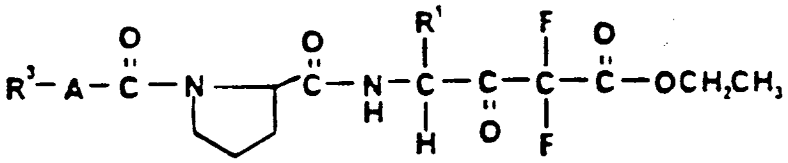

XXVIb

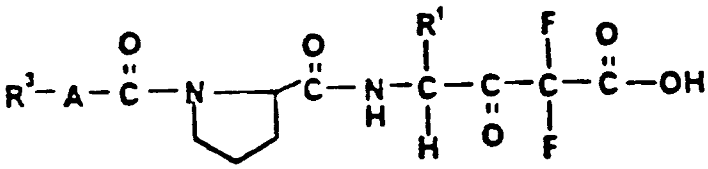

XXVIIb

## Claims

1. A compound of the formula Ib:

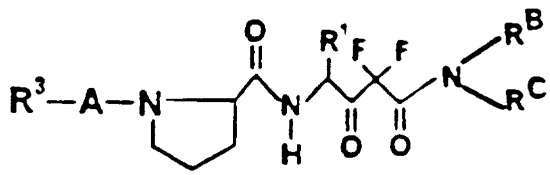

Ib

wherein $R^1$ is (1-3C) alkyl; $R^3$ is benzyl; $R^C$ is hydrogen; A is oxycarbonyl; and $R^B$ is selected from $-CH(CH_2C_6H_5)COOCH_3$, $-CH(CH_2C_6H_5)CONH_2$, $-CH(CH_2OH)CONH.CH(CH_2C_6H_5).(CONH_2)$, $-CH(CH_2C_6H_5)COOH$, $-CH_2COOCH_3$, $-CH_2COOH$, $-CH(CH_2C_6H_5)CONH.CH_2COOCH_3$, $-CH(CH_2C_6H_5)CONH.CH_2COOH$, $-CH[(CH_2)_4NHOCOCH_2C_6H_5]COOCH_2C_6H_5$, $-CH[(CH_2)_4NHOCOCH_2C_6H_5]COOH$, $-CH[(CH_2)_2OCOCH_2C_6H_5]COOCH_2C_6H_5$, $-CH(CH_2C_6H_5)COOCH_2C_6H_5$ and $-CH_2COOCH_2C_6H_5$; or a pharmaceutically acceptable salt thereof.

2. A compound as claimed in claim 1 wherein $R^1$ is isopropyl.

3. A compound of the formula Ib as set out in claim 1 wherein $R^1$ is isopropyl; $R^3$ is benzyl; A is oxycarbonyl; $R^C$ is hydrogen; and $R^B$ is selected from:

(a) a group of formula IIa

-CH($R^6$).CONH.CH($R^7$).CO.$R^8$ IIa

in which $R^6$ is hydroxymethyl or benzyl; $R^7$ is hydrogen or benzyl; and $R^8$ is amino, methoxy or hydroxy; and

(b) a group of formula IIb

-CH($R^6$).CO.$R^8$ IIb

in which $R^6$ is hydrogen, benzyl or hydroxymethyl; and $R^8$ is amino, methoxy, hydroxy or benzyloxy; or a pharmaceutically acceptable salt thereof.

**4.** A compound as claimed in claim 1, 2 or 3 wherein a chiral centre at $R^1$ has an S configuration.

**5.** A salt as claimed in any one preceding claim which is selected from alkali metal, alkali earth metal and organic amine salts, and from salts with acids forming pharmaceutically acceptable acid addition salts.

**6.** A pharmaceutical composition comprising a compound of the formula I, or a pharmaceutically acceptable salt thereof, as defined in any one preceding claim, together with a pharmaceutically acceptable diluent or carrier.

**7.** A process for producing a compound of the formula Ib as defined in claim 1, or a pharmaceutically acceptable salt thereof, which is characterised by:

(a) oxidizing a compound of the formula VIIIb

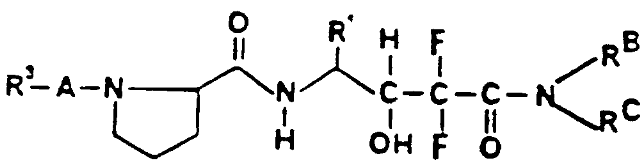

VIIIb

or

(b) coupling an acid of formula XXVIIb

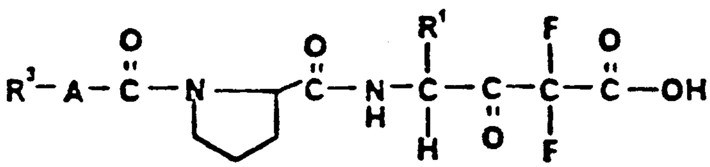

XXVIIb

with an amine of formula V

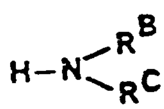

V

or

(c) for a compound of the formula Ib wherein $R^B$ contains a carboxy group, decomposing the ester group of a corresponding compound of formula Ib in which $R^B$ contains an ester group;

whereafter, when a salt is desired, a compound of formula Ib is reacted with a suitable acid or base; and wherein $R^1$, $R^3$, $R^C$, A and $R^B$ have any of the meanings defined in claim 1.

## Patentansprüche

1. Verbindung mit der folgenden Formel Ib:

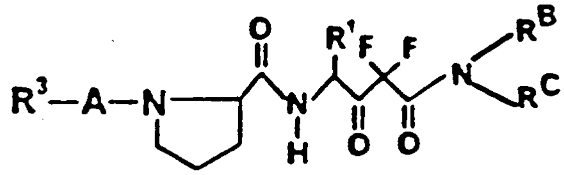

Ib

oder ein pharmazeutisch geeignetes Salz davon, in der $R^1$ für (1-3C)-Alkyl steht, $R^3$ für Benzyl steht, $R^C$ für Wasserstoff steht, A für Oxycarbonyl steht, und $R^B$ unter $-CH(CH_2C_6H_5)COOCH_3$, $-CH(CH_2C_6H_5)$-$CONH_2$, $-CH(CH_2OH)CONH.CH(CH_2C_6H_5).(CONH_2)$, $-CH(CH_2C_6H_5)COOH$, $-CH_2COOCH_3$, $-CH_2COOH$, $-CH(CH_2C_6H_5)CONH.CH_2COOCH_3$, $-CH(CH_2C_6H_5)CONH.CH_2COOH$, $-CH[(CH_2)_4NHOCOCH_2C_6H_5]$-$COOCH_2C_6H_5$, $-CH[(CH_2)_4NHOCOCH_2C_6H_5]COOH$, $-CH[(CH_2)_2OCOCH_2C_6H_5]COOCH_2C_6H_5$, -CH-$(CH_2C_6H_5)COOCH_2C_6H_5$ und $-CH_2COOCH_2C_6H_5$ ausgewählt ist.

2. Verbindung nach Anspruch 1, in der $R^1$ für Isopropyl steht.

3. Verbindung mit der Formel Ib nach Anspruch 1 oder ein pharmazeutisch geeignetes Salz davon, in der $R^1$ für Isopropyl steht, $R^3$ für Benzyl steht, A für Oxycarbonyl steht, $R^C$ für Wasserstoff steht und $R^B$ ausgewählt ist unter:
   (a) einer Gruppe mit der Formel IIa

   $-CH(R^6).CONH.CH(R^7).CO.R^8$ IIa

   in der $R^6$ für Hydroxymethyl oder Benzyl steht, $R^7$ für Wasserstoff oder Benzyl steht und $R^8$ für Amino, Methoxy oder Hydroxy steht, und
   (b) einer Gruppe mit der Formel IIb

   $-CH(R^6).CO.R^8$ IIb

   in der $R^6$ für Wasserstoff, Benzyl oder Hydroxymethyl steht und $R^8$ für Amino, Methoxy, Hydroxy oder Benzyloxy steht.

4. Verbindung nach Anspruch 1, 2 oder 3, bei der ein Chiralitätszentrum an $R^1$ die S-Konfiguration aufweist.

5. Salz nach einem der vorhergehenden Ansprüche, das unter Alkalimetall-, Erdalkalimetall- und organischen Aminsalzen und unter Salzen mit Säuren, die pharmazeutisch geeignete Säureadditionssalze bilden, ausgewählt ist.

6. Pharmazeutische Zusammensetzung, die eine in einem der vorhergehenden Ansprüche definierte Verbindung mit der Formel I oder ein pharmazeutisch geeignetes Salz davon zusammen mit einem pharmazeutisch geeigneten Streckmittel oder Trägermittel enthält.

7. Verfahren zur Herstellung einer in Anspruch 1 definierten Verbindung mit der Formel Ib oder eines pharmazeutisch geeigneten Salzes davon, dadurch gekennzeichnet, daß
   (a) eine Verbindung mit der folgenden Formel VIIIb

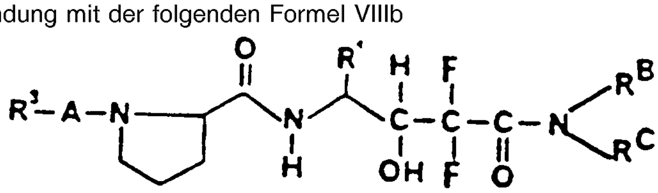

VIIIb

oxidiert wird oder

(b) eine Säure mit der folgenden Formel XXVIIb

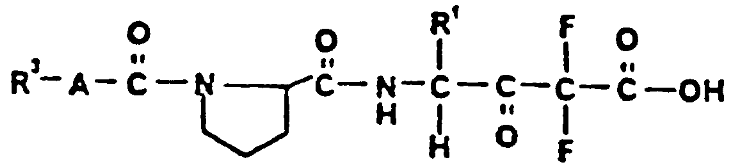

XXVIIb

mit einem Amin mit der folgenden Formel V

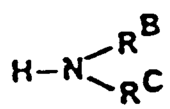

V

gekuppelt wird oder

c) für eine Verbindung mit der Formel Ib, in der $R^B$ eine Carboxy-Gruppe enthält, die Ester-Gruppe einer entsprechenden Verbindung mit der Formel Ib, in der $R^B$ eine Ester-Gruppe enthält, zersetzt wird,

woraufhin, wenn ein Salz gewünscht wird, eine Verbindung mit der Formel Ib mit einer geeigneten Säure oder Base umgesetzt wird, wobei $R^1$, $R^3$, $R^C$, A und $R^B$ jede der in Anspruch 1 definierten Bedeutungen haben.

## Revendications

1. Composé de formule Ib :

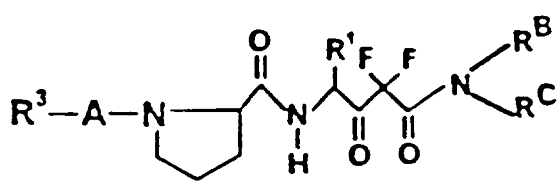

Ib

dans laquelle $R^1$ est un groupe alkyle en $C_1$ à $C_3$ ; $R^3$ est un groupe benzyle ; $R^C$ est de l'hydrogène ; A est un groupe oxycarbonyle; et $R^B$ est choisi entre les groupes $-CH(CH_2C_6H_5)COOCH_3$, $-CH(CH_2C_6H_5)CONH_2$, $-CH(CH_2OH)CONH.CH(CH_2C_6H_5).(CONH_2)$, $-CH(CH_2C_6H_5)COOH$, $-CH_2COOCH_3$, $-CH_2COOH$, $-CH(CH_2C_6H_5)CONH.CH_2COOCH_3$, $-CH(CH_2C_6H_5)CONH.CH_2COOH$, $-CH[(CH_2)_4NHOCOCH_2C_6H_5]COOCH_2C_6H_5$, $-CH[(CH_2)_4NHOCOCH_2C_6H_5]COOH$, $-CH[(CH_2)_2OCOCH_2C_6H_5]COOCH_2C_6H_5$, $-CH(CH_2C_6H_5)COOCH_2C_6H_5$ et $-CH_2COOCH_2C_6H_5$ ; ou un sel pharmaceutiquement acceptable de ce composé.

2. Composé suivant la revendication 1, dans lequel $R^1$ est un groupe isopropyle.

3. Composé de formule Ib suivant la revendication 1, dans lequel $R^1$ est un groupe isopropyle ; $R^3$ est un groupe benzyle ; A est un groupe oxycarbonyle ; $R^C$ est de l'hydrogène ; et $R^B$ est choisi entre :

(a) un groupe de formule IIa

$-CH(R^6).CONH.CH(R^7).CO.R^8$ IIa

dans laquelle $R^6$ est un groupe hydroxyméthyle ou benzyle ; $R^7$ est de l'hydrogène ou un groupe benzyle ; et $R^8$ est un groupe amino, méthoxy ou hydroxy ; et

(b) un groupe de formule IIb

-CH($R^6$).CO.$R^8$ IIb

dans laquelle $R^6$ est de l'hydrogène, un groupe benzyle ou hydroxyméthyle ; et $R^8$ est un groupe amino, méthoxy, hydroxy ou benzyloxy ;

ou un sel pharmaceutiquement acceptable de ce composé.

**4.** Composé suivant la revendication 1, 2 ou 3, dans lequel un centre de chiralité au niveau de $R^1$ a la configuration S.

**5.** Sel suivant l'une quelconque des revendications précédentes, qui est choisi entre des sels de métaux alcalins, de métaux alcalino-terreux et d'amines organiques, et des sels d'acides formant des sels d'addition d'acides pharmaceutiquement acceptables.

**6.** Composition pharmaceutique, comprenant un composé de formule I ou un sel pharmaceutiquement acceptable de ce composé tel que défini dans l'une quelconque des revendications précédentes, en association avec un diluant ou support acceptable du point de vue pharmaceutique.

**7.** Procédé de production d'un composé de formule Ib suivant la revendication 1 ou d'un sel pharmaceutiquement acceptable de ce composé, qui est caractérisé par :

(a) l'oxydation d'un composé de formule VIIIb

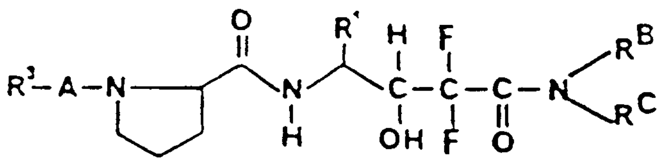

VIIIb

ou

(b) le couplage d'un acide de formule XXVIIb

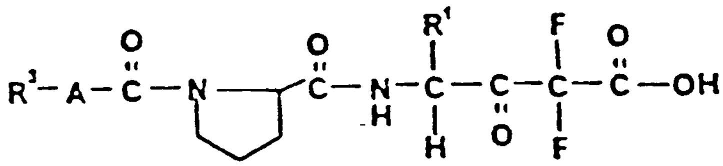

XXVIIb

avec une amine de formule V

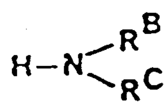

V

ou

(c) pour un composé de formule Ib dans laquelle $R^B$ contient un groupe carboxy, la décomposition du groupe ester d'un composé correspondant de formule Ib dans laquelle $R^B$ contient un groupe ester ;

après quoi, lorsqu'on désire un sel, on fait réagir un composé de formule Ib avec un acide ou une base convenable ; et dans laquelle $R^1$, $R^3$, $R^C$, A et $R^B$ ont l'une quelconque des définitions données dans la revendication 1.